Europäisches Patentamt

European Patent Office

Office européen des brevets

(19)

(11) Publication number: **0 278 160**
**A2**

# EUROPEAN PATENT APPLICATION

(21) Application number: 87310179.4

(22) Date of filing: **18.11.87**

(51) Int. Cl.4: **C12P 21/00** , **C12N 15/00** ,
**G01N 33/577**

The applicant has filed a statement in accordance with Rule 28 (4) EPC (issue of a sample only to an expert). Accession number(s) of the deposit(s): ECACC 86111001.

The title of the invention has been amended (Guidelines for Examination in the EPO, A-III, 7.3).

(30) Priority: **19.11.86 DK 5549/86**

(43) Date of publication of application:
**17.08.88 Bulletin 88/33**

(84) Designated Contracting States:
**AT BE CH DE ES FR GB GR IT LI LU NL SE**

(71) Applicant: **NOVO INDUSTRI A/S**
**Novo Allé**
**DK-2880 Bagsvaerd(DK)**

(72) Inventor: **Kjeldsen, Thomas Borglum**
**Gronnevej 87**
**DK-2830 Virum(DK)**
Inventor: **Zeuthen, Jesper**
**Peder Godskesvej 8**
**DK-2830 Virum(DK)**

(74) Representative: **Raynor, John et al**
**W.H. Beck, Greener & Co 7 Stone Buildings**
**Lincoln's Inn**
**London WC2A 3SZ(GB)**

(54) **Human monoclonal anti cancer antibodies, hybridoma cell lines producing them and their uses.**

(57) Human-human hybrid cell lines producing monoclonal antibodies against antigenic determinants on cancer cells, particularly carcinomas of the breast and colon.

The antibodies are useful in the diagnosis and treatment of especially mammary carcinoma.

EP 0 278 160 A2

0 278 160

## ANTIBODY

FIELD OF THE INVENTION

This invention relates to human-human hybrid cell lines that produce monoclonal antibodies against antigenic determinants on cancer cells, particularly carcinoma of the breast and colon.

BACKGROUND OF THE INVENTION

In the past few years there has been considerable research effort focused on developing immunotherapeutic regimes for treating cancer. In nearly all of these studies antibodies against tumor-associated antigens have been utilized to treat patients suffering from various malignant disorders, unfortunately, with little success. There appear to be four major reasons for the lack of success, two being: first, tumor-associated antigens are difficult to identify; and, second, it is technically difficult and laborious to prepare homogeneous antibody that recognize tumor-associated antigens. The latter difficulty has been largely circumvented by the development of the hybridoma technique of Kohler and Milstein (Nature, 256, p.495-497, 1975), which allows for the unlimited production of monoclonal antibody. As for identifying tumor-associated antigens, there is at present no sure way to identify antigens restricted to cancer cells.

The third problem which must be surmounted when devising an immunotherapeutic regime for treatment of cancer is to prevent an immune reaction against the immunotherapeutic agent, that is, against the antibodies directed to the tumor-associated antigen. This problem is amenable to solution by employing human antibodies generated by modification of the basic Kohler and Milstein technique, as described by Kozbor and Roder (Immunol.Today 4 (1983) 72-79), Glassy et al. in Monoclonal Antibodies and Cancer, eds.Boss et al (1983), Academic Press, and Glassy et al, Proc. Natl.Acad.Sci. USA 80:6327 (1983). Human monoclonal antibodies, when injected into a patient bearing a tumor, recognize and bind to the tumor by binding to the tumor-associated antigen. Since the antibody is of human origin it will not be "seen" as a foreign substance by the patient's immune system and is therefore immunologically blind.

Fourth, most of the human monoclonal antibodies generated to date are of the IgM class. This class of antibody, although useful for a variety of in vitro studies, is not as clinically useful as antibody of the IgG class. The generation of one class of monoclonal antibody over another is, at present, poorly understood, and hence not reproducible.

Unfortunately, the history of human hybridomas has been marked by substantial and often exasperating technical problems, and the first report of hybrids secreting human immunoglobulins of "desired" specificity did not appear until 1980. It soon became clear that, while it was possible to fuse human lymphocytes, the frequency of fusion, level of Ig synthesis and their poor stability implied that production of human antibodies by the hybridoma technique was neither routine nor yet a practical approach. However, during the past 2-3 years, some progress has been made. Relatively efficient B-lymphocyte fusion partners have been developed and the methods of Epstein-Barr virus (EBV) transformation improved. In the following, methods and problems encountered in the preparation of human monoclonal antibodies will be discussed.

Human monoclonal antibodies can be prepared by interspecies hybridization. The lack of suitable human fusion partners prompted attempts to produce human monoclonal antibodies by hybridization of human lymphocytes to a non-human fusion partner. With interspecies hybridomas it was possible to prepare monoclonal antibodies reacting with tetanus toxin (Kozbor et al., Hybridoma 1 (1982) 323-328), keyhole limpet hemocyanin (Lane et al., J.Exp.Med. 155 (1982) 333-337) and various human cancers. Interspecies hybridomas lose human rather than murine chromosomes (Cote et al., Feder.Proc. 43 (1984) 2456-2459; Kozbor & Roder, Immunol.Today 4 (1983) 72-79) and the loss of human chromosomes is not random. The loss of chromosome 14 (heavy chain) and chromosome 22 ($\lambda$ chain) is less frequent than the loss of chromosome 2 ($x$ -chain) (Erikson et al., Nature 294 (1981) 173-175).

Intraspecies human hybridomas are more stable in their chromosome composition than analogous interspecies hybridomas (Kozbor & Roder, Immunol.Today 4 (1983) 72-79). Therefore, development of human/human hybridomas is a likely source of human monoclonal antibodies. Lack of suitable human fusion partners has been a major obstacle to this approach. Human myelomas are difficult to culture in vitro, they tend to fuse with a low frequency and are difficult to render resistant to DNA analogues. Most myeloma-derived human fusion partners have a doubling time of 36-70 h and need myeloma-stimulating factors to proliferate in vitro. Morphologically, myelomas are characterized by abundant rough endoplasmatic reticulum (RER), few free polyribosomes, abundant mitochondria and a well-developed Golgi apparatus. The level of immunoglobulin secretion is high (Kozbor et al., in Human Hybridomas and Monoclonal Antibodies, Engleman et al (eds) Plenum Press, N.Y. (1985) 21-36).

Human lymphoblastoid cell lines (LCL) grow easily in vitro and are used as alternative fusion partners. LCL grow faster than myeloma cell lines and are always associated with epstein-Barr virus. Morphologically, LCL are characterized by poorly developed RER and Golgi apparatus, many free polyribosomes, and a low level of immunoglobulin secretions (Kozbor et al., supra).

Generally, both human myeloma cells and LCL are suboptimal as fusion partners for generation of human hybridomas. Work with human hybridomas has been hampered by the lack of optimal human fusion partners. Human lymphoblastoid cell lines appear to be more efficient fusion partners than human myelomas. Human hybridomas generated with LCL tend to produce less immunoglobulin than human hybridomas generated with myelomas as fusion partners. During the past few years, progress has been made with the preparation of better human fusion partner by use of recombinant DNA technology and somatic cell fusion techniques. for instance, Teng et al., Proc.Natl.Acad.Sci. USA 80 (1983) 7308-7312 constructed mouse/human heteromyeloma fusion partners.

Another example is the construction by Kozbor et al., J.Immunol. 133 (1985) 3001-3005 of the KR-12 cell line by fusion of an ouabain-resistant variant of the GM 1500 6TG-2 lymphoblastoid cell line (KR-4) with human myeloma cells (RPMI 8226). The KR-12 cell line was designed for fusion with EBV-transformed lymphoblastoid cell lines in the EBV-hybridoma technique. The conclusion to be drawn from these two examples is that engineering of new cell lines as fusion partners will augment the fusion efficiency, which has been one of the main obstacles in the preparation of human monoclonal antibodies.

The third method for production of human monoclonal antibodies is the EBV-hybridoma technique. EBV is a lymphotropic herpes virus, which transforms normal B-lymphocytes and makes it possible to culture these cells as permanent lines. Clones producing immunoglobulins of interest can be isolated and then fused with a human fusion partner with a dominant marker to stabilize and amplify the production of immunoglobulins. The EBV-hybridoma technique is very effective with certain types of antigens.

At present immunotherapy has been of little use in treating or diagnosing cancers of the breast, colon, vulva, stomach or other organs for the above mentioned reasons. Thus the establishment of human-human hybrid cell lines that secrete monoclonal antibodies against tumor-associated antigens is sorely needed.

SUMMARY OF THE INVENTION

According to this invention, human-human hybrid cell lines, i.e., hybridomas, that synthesize and secrete human IgM monoclonal antibodies are generated by fusing lymphocytes isolated from an axillary draining lymph node from a patient suffering from carcinoma of the breast to a drug-resistant human hybrid cell line.

DETAILED DESCRIPTION OF THE INVENTION

In accordance with the present invention B cell lymphocytes are isolated from a patient that exhibits a tumor burden. The B cells are isolated by surgical techniques from lymph nodes of the patient, particularly from axillary draining lymph nodes near the tumor mass. Alternatively, B cells can be isolated from other lymphoid organs, such as spleen, tonsils and peripheral blood. Regardless of the organs used to obtain the B cells, the latter are prepared in a form suitable for hybridoma formation. Generally, this consists in following the procedures described by Schlom et al., as decribed in the Proceedings of the National Academy of Sciences, USA, vol 77, p. 6841 (1980), and Sikora et al. as described in the British Jornal of Cancer, vol. 43, p. 698 (1981), and by Glassy et al., Proc.Natl.Acad.Sci. USA 80:6327 (1981). These procedures are hereby incorporated by reference. This involves obtaining lymph nodes from patients within few hours after they are removed by surgery. Homogenizing them in Roswell Park Memorial Institute 1640 (RPMI-1640) media to release the lymphocytes.

In order to obtain human hybrids the B lymphocytes must be fused to a human cell line that exhibits a drug selectable marker; generally such cell lines are of lymphoblastoid origin or a hybrid cell between a lymphoblastoid cell and a myeloma cell. Alternatively the human cell line could be selected based on its ability to exhibit temperature sensitive inhibition of growth. The cell line is maintained in RPMI-1640 culture media prior to fusion.

To form human-human hybrid cell* lines the lymph node lymphocytes are mixed with the lymphoblastoid or hybrid cell; usually an equal or ten-fold greater number of lymph node lymphocytes are combined with the cell line. The two cell types are pelleted to the bottom of a centrifuge tube and fused for an appropriate time with a chemical fusing agent, particularly polyethylene glycol (PEG). Alternatively, fusion can be accomplished by electro cell fusion or by viruses, particularly Sendai and Abelson. Fused cells are then plated in media containing drugs which selectively kill unfused cells. The hybrid cell lines which grow up, generally within 10 or 20 days, are screened for human antibody production by assaying for antibodies present in the culture media using one or many immunoassay methods, but particularly that described below.

It will be apparent to those skilled in the art that the genes that encode antibody in the hybridoma cell lines can, by DNA recombinant techniques, be transferred to other cell types, and that the latter can act as a source of monoclonal antibody.

Monoclonal antibodies present in the culture media of the hybridomas were purified by standard techniques, including exclusion and affinity chromotographic procedures and were assayed to determine which immunoglobulin class they belong to, and their cell type specificities. Antibody class determination was conducted by ELISA techniques using the appropriate antisera and found to be of the IgM and IgG classes as described below. To determine the cell type specificity both normal and cancer tissue was assayed on frozen sections and formalin fixed parafine embedded sections using an avidin-biotin system (ABC kit, Vector Labs.).

A stable subclone of the cell line MAC 40/43 is on deposit at the European Collection of Animal Cell Cultures (ECACC) PHLS Centre for Applied Microbiology and Research, Porton Down, Salisbury, Wiltshire SP4 0JG , UK, for the purposes of patent procedure on the date indicated below. ECACC being an international depositary authorized under the Budapest Treaty, affords permanence of the deposit in accordance with rule 9 of the Treaty.

Deposit date: 10 November 1986

Depositors reference: Hybridoma cell line MAC 40/43

ECACC designation: 86 11 10 01

The monoclonal antibodies of the invention selectively react with non-haematopoietic human cancers, particularly tumors of the breast and testes and to a lesser degree with tumors of the skin, bladder and colon. It is important to note that while the monoclonal antibodies have been found to react only with the aforementioned tumors, it is to be anticipated that they will react with other tumors that express the antigens recognized by the monoclonal antibodies.

Monoclonal antibodies generated as described above are expected to inhibit the growth of tumor cells in vitro. This would be accomplished by adding monoclonal antibodies in the range of 5 - 50 $\mu$g/ml to tumor cells and measuring their growth rate over several days. It is to be anticipated that the monoclonal antibodies generated by this invention will inhibit the growth of a wide variety of other tumor cell types, and furthermore that fragments, or combinations of antibody heavy and light chains will inhibit tumor growth as the antigen combining site of the molecule is retained.

The example below illustrates the invention. However, it is to be understood that various changes and modifications may be made without departing from the spirit of the invention.

## EXAMPLE

### Cell culture medium.

Human fusion partners and human hybridomas were grown in RPMI-1640 (Gibco, UK), supplemented with 2 mM glutamine, 50 $\mu$g streptomycin per ml, 50 units penicillin per ml, 3.5x10$^{-5}$ M 2-mercaptoethanol (Sigma, USA) and 10-15% heat inactivated fetal calf serum (FCS) (all from Gibco, UK), in the following designated complete RPMI. HAT medium consisted of complete RPMI supplemented with 13.6 $\mu$g hypoxanthine per ml, 0.18 $\mu$g aminopterin per ml and 3.9 $\mu$g thymidine per ml (all from Gibco, UK). HT medium consisted of complete RPMI supplemented with 13.6 $\mu$g hypoxanthine per ml and 3.9 $\mu$g thymidine per ml. The human breast cancer cell line MCF-7 was grown in complete RPMI containing 5% fetal calf serum and cells were detached with trypsin-EDTA (Gibco, UK).

0 278 160

## Cell line.

The human fusion partner KR-12 (Kozbor, D., Tripputi, P., roder, J.C. & Croce, C.M. (1984) J. Immunol. 133, 3001-3005) is a hybrid between the human myeloma RPMI-8226 (a producer of λ -chains) and the human lymphoblastoid cell line KR-4 (a producer of γ2 and x -chains), which is an ouabain-resistant mutant of the human B-lymphoblastoid cell line GM 1500. The KR-12 cell line is phenotypically similar to human myeloma cells and proliferates like the lymphoblastoid cell line KR-4. KR-12 secretes γ2, λ and x -chains. KR.12 was obtained from the Wistar Institute, Philadelphia, USA.

## Hybridoma techniques.

Segments of sterile human axillary lymph nodes were obtained from breast cancer patients undergoing mastectomy. Fat was removed from the nodes and a single cell suspension was prepared by homogenization. The suspended lymph node cells were washed 3 times in RPMI-1640.

Human lymph node cells and the human fusion partner in the log phase of growth were mixed in ratios of 1:1 or 2:1 and fused with polyethylene glycol 4000 (PEG) (50% w/v in RPMI 1640) (Merck, W.Germany) as described by Köhler and Milstein (1975) above and modified by Gefter et al. (Gefter, M.L., Marquiles, D.M. & Scharff, M.D. (1977) Somatic Cell Genet. 3, 231-236). 1 ml of PEG heated to 37°C was slowly added to the cell pellet over a period of 1 min, the cells were mixed gently for 1.5 min and 10 ml of RPMI 1640 was added drop-wise over a period of 5 min. The cells were allowed to stand for 10 min before the cell suspension was washed 3 times with RPMI-1640, resuspended ($5 \times 10^5$ cells per ml) and seeded in wells on 3 to 5 microtiter plates (Costar Plastics, USA), depending on the total number of cells. Cells were seeded diretly in 200 μl HAT medium. 100 μl of medium per well was removed twice a week and replaced with 100 μl fresh medium. Cells were maintained in HAT medium for 3 weeks and the medium was gradually changed to HT medium. When hybridomas grew vigorously, the medium was changed to complete RPMI.

Hybridomas usually appeared between weeks 3 and 4 after fusion. Hybridoma culture supernatants were subsequently tested for the presence of human immunoglobulins. Hybridoma cultures producing human immunoglobulins (>1 μg/ml) were transferred to 1.5 ml wells (multidish 24 plates, NUNC, Denmark). Hybridoma culture supernatants containing immunoglobulins were assayed for reactivity with the human breast cancer cell line MCF-7 by cell-binding ELISA and with autologous breast tumor tissue by immunohistochemistry. Hybridomas producing antibodies reacting with either MCF-7, or autologous breast tumor, or with both, were transferred to 50 ml tissue culture bottles (NUNC, Denmark) and viable aliquots were frozen in 50% v/v PCS, 40% v/v and 10% v/v dimethyl sulfoxide (Sigma, USA).

The hybridomas were cloned under standard conditions by limiting dilution at densities of 10 cells/well and 1 cell/well. peritoneal macrophages from one BALB/c mouse were distributed in each microtiterplate as feeder cells. 8-10 days after cloning, the wells were examined by microscopy for single clone growth. When clones had grown to 40-50% confluence, typically 2 to 4 weeks after cloning, the supernatant from the wells containing one hybridoma colony was harvested and assayed for the presence of human immunoglobulins reacting with human breast cancer cells. Selected clones were transferred to 24-well plates for expansion, after which cloning was repeated. hybridomas were cloned at least twice.

The results of these experiments are expressed in table I below.

5

## 0 278 160

Table 1. Generation of human hybridomas with lymphocytes from axillary lymph nodes from breast cancer patients.

| Patient | Fusion | Hybri-domas | Hyb. Ig | | | MCF-7/Tumor reactive MAb |
|---|---|---|---|---|---|---|
| | | | M | G | G/M | |
| 1 | 1 | 0 | | | | |
| 2 | 2 | 48 | 5 | 13 | 3 | 1 (2.0%)[x] |
| 3 | 3 | 0 | | | | |
| 4 | 4 | 6 | 2 | 1 | | |
| 5 | 5 | 3 | | 1 | | |
| 6 | 6 | 0 | | | | |
| 7. | 7 | – | | | | |
| 8 | 8 | – | | | | |
| 9 | 9 | 40 | 4 | 1 | 4 | |
| 10 | 10 | 199 | 32 | 73 | 4 | 40 (20.1%) |
| 11 | 11 | 52 | 31 | 12 | 3 | 9 (17.3%) |
| | | 348 | 74 | 101 | 14 | 50 (14.4%) |

Hyb. Ig = Hybridomas producing more than 1 µg human IgM or/and IgG per ml supernatant.

MAb = monoclonal antibody.

x = % tumor-reactive antibodies.

From table I it is seen that 11 human/human fusions were performed with lymph node cells from 11 patients. A total of 348 human hybridomas were obtained, 189 (50.3%) of which produced human immunoglobulin. 50 of the human hybridomas initially produced human immunoglobulin reactive either with MCF-7 cells, with autologous tumor tissue, or with both. These monoclonal antibodies were derived from 3 of the 11 patients. Once established, the human hybridomas showed rapid growth, with doubling times of 24-36 h.

## Quantitation of human immunoglobulins by ELISA.

Human immunoglobulins in hybridoma culture supernatants were measured by ELISA. The ELISA used was a modification of the method described by Douillard and Hoffman in Methods in Enzymology, eds. Langone, J.J. & van Vunakis, H. (Academic Press, Inc., New York), 92, pp.168-174. 50 µl of rabbit anti-human IgG antibody (DAKO, Denmark) diluted 1:1000 in PBS was distributed in a 96-well, flat-bottomed immunoplate (NUNC, Denmark) and the plate was incubated 1 h at 22°C. After 3 successive washes with PBS-T, 200 µl PBS-BSA was added to each well and incubated for 1 h at 22°C. After 3 successive washes with PBS-T, 50 µl of hybridoma supernant was added to each well and incubated for 1 h at 22°C. After 3 successive washes with PBS-T, 50 µl of peroxidase-conjugated rabbit anti-human IgG antibody (DAKO, Denmark) diluted 1:1000 in PBS-BSA was added to each well and allowed to incubate for 1 h at 22°C. After 3 successive washes with PBS-T, 100 µl of o-phenylendiamine (Merck, W.Germany), 0.5 mg/ml in 50 mM citrate/phosphate buffer, pH 5.3, containing 0.015% hydrogen peroxide (Merck, W.Germany) was added to each well and the plates were allowed to stand for 3-5 min before the enzyme reaction was terminated by adding 150 $\mu$l 2N H$_2$SO$_4$ per well. The absorbance was determined on an ELISA reader (Kontron SLT 210, SLT Labinstruments). The concentration of immunoglobulin was calculated by interpolation on a standard curve constructed using a human serum reference. ELISA for IgM, $x$ and $\lambda$-chains were performed in the same way, using the appropriate antibodies (all from DAKO, Denmark).

Cell-binding ELISA.

The reactivity of human monoclonal antibodies with MCF-7 fixed in glutaraldehyde was determined by cell-binding ELISA. Cells were washed 3 times in PBS, suspended in PBS at 1.0x10$^6$ cells per ml and distributed (50 $\mu$l per well) in flat-bottom immunoplates pretreated with 0.001% polyL-lysin (Sigma, USA) in PBS. the plates were centrifuged at 100 x g for 5 min, 50 $\mu$l of 0.5% glutaraldehyde (Sigma, USA) in PBS was added to each well and the plates were allowed to stand for 15 min at 22°C. Free glutaraldehyde was removed by 3 washes with PBS-T. 200 $\mu$l PBS-BSA containing 0.1 M glycine was added to each well and the plates were incubated for 1 h at 22°C. 50 $\mu$l of hybridoma supernatant was added to each well and allowed to react for 1 h at 22°C. After 3 washes, 50 $\mu$l of peroxidase-conjugated goat anti-human Ig antibody (Tago, USA) diluted 1:3000 in PBS-BSA was added to each well and the plates were incubated for 1 h at 22°C. After another 3 washes, 100 $\mu$l enzyme substrate was added to each well and the enzyme reaction terminated with H$_2$SO$_4$ after 5-15 min. A reaction was considered positive when the absorbance was three times the absorbance with KR-12 cell line immunoglobulin in a similar concentration.

Hybidoma culture supernatants were tested 3-5 weeks after the fusion for production of human IgG and IgM. Of the 348 wells containing human hybridomas, 189 wells contained hybridomas producing 1 $\mu$g human immunoglobulin per ml supernatant. 74 wells contained hybridomas producing IgM, 101 wells contained hybridomas producing IgG and 14 wells contained hybridomas producing both IgM and IgG.

20 human hybridomas producing antibodies with strong reactivity with either MCF-7 or autologous tumor tissue were selected for stabilization of immunoglobulin production. 8 of these human hybridomas were cloned to 100% stability.

Once stabilized, the human hybridomas were found to remain stable, producing human immunoglobulin in the range of 4.0 to 17.0 $\mu$g per ml supernatant under normal growth conditions (i.e. in complete RPMI-1640 containing 10% FCS, without feeder cells and with a twice-weekly change of medium); the lower hybridoma density was 5x10$^4$ hybridomas per ml and the upper density was 1x10$^6$ hybridomas per ml.

The 8 hybridomas that were cloned to 100% stability were characterized further, and the results are indicated in table II.

Table 2. Characterization of human monoclonal antibodies
reacting with human breast tumors

| Antibody | $\mu Ig/ml$[x) | Reaction with MCF-7 cells[+) | ELISA for HC[++) | ELISA for LC[++) | PAGE[o] |
|---|---|---|---|---|---|
| MAC 37/16 | 7.5 | no | $\gamma$ | $\kappa$ | $\gamma$, LC |
| MAC 40/43 | 17.0 | yes | $\gamma + \mu$ | $\lambda$ | $\gamma$, $\mu$, LC |
| MAC 37/8 | 7.5 | no | $\gamma$ | $\kappa$ | $\gamma$, LC |
| MAC 37/135 | 6.0 | yes | $\gamma + \mu$ | $\kappa$ | $\gamma$, $\mu$, LC |
| MAC 37/121 | 5.0 | (yes) | $\gamma$ | $\lambda$ | $\gamma$, LC |
| MAC 37/102 | 8.0 | no | $\gamma$ | $\kappa$ | $\gamma$, LC |
| MAC 14/39 | 16.0 | yes | $\gamma$ | $\lambda$ | $\gamma$, LC |
| MAC 37/69 | 4.0 | no | $\gamma$ | $\kappa$ | $\gamma$, LC |

x) determined by ELISA

+) performed with hybridoma culture supernatant

xx) HC:  heavy chain KR-12; secretes $\gamma$, $\lambda$, $\kappa$

++) LC:  light chain UC729HF$_2$ secretes $\kappa$

o) PAGE:  polyacrylamide gel electrophoresis

Immunohistology.

The initial screening for reactivity was performed on 5 μM sections of freshly frozen autologous tumor tissue sliced on a cryostat. The sections were preincubated for 30 min. with normal goat serum, washed in TBS and incubated overnight at 4°C with undiluted supernatant from antibody producing hybridomas. The sections were then rinsed in TBS and incubated 45 min with goat anti-human IgG or IgM (depending on the subclass of the primary antibody) diluted 1:3000. After another rinse the sections were incubated for 30 min with 1:100 peroxidase conjugated swine antigoat IgG. the reaction was developed with AEC, the sections were counterstained with haematoxylin and mounted in Aquamount.

In the case of a positive reaction, the antibodies were applied to sections of formalin-fixed, paraffin-embedded tissue, and only such antibodies where the corresponding epitope was preserved despite fixation were included in further screening.

To reduce the background staining and to enhance the specific reaction, the above three-layer method was substituted with an avidin-biotin system. Biotinylated primary antibody was applied to the deparaffinized section for 30 min, followed by washing in PBS and application of an avidin-biotin system (ABC kit, Vector labx.). The positive reaction was developed with AEC, the sections were counterstained with haematoxylin and mounted in Aquamount.

As already mentioned, the initial screening was performed on autologous breast carcinoma. The specificities of the antibodies selected on this basis were tested on a wide range of different tissue types of both epithelial, mesenchymal and lymphoid origin. The reaction in breast tissue was investigated in 150 primary breast carcinomas, 19 benign breast tumors and 9 cases of normal breast tissue.

A positive antigen-antibody reaction was characteristically seen as a reddish-brown, intracytoplasmically located colouration. There was no tendency toward a preferential apical staining in tubular structures. The nuclei were always negative. The specificity of the reaction of some of the antibodies is illustrated in table III.

Table 3. Reaction patterns of 5 different human monoclonal antibodies   .

| Human MAb | % pos. breast car- cinomas | Breast tissue | | Epithelium | | Mesenchymal tissue | | Lymphoid | |
| | | normal | benign tumor | normal | malig- nant | normal | malignant | normal | malig- nant |
|---|---|---|---|---|---|---|---|---|---|
| MAC 40/43 | 59 | – | (+) | (+) | + | – | – | – | – |
| MAC 14/39 | 89 | n.d. | n.d.[x] | + | + | + | + | – | – |
| MAC 37/16 | 35 | + | + | + | + | (–) | + | – | – |
| MAC 37/121 | 15 | + | + | + | + | (–) | + | – | – |
| MAC 37/102 | – | + | + | + | + | (–) | + | – | – |

x n.d.: not determined

0 278 160

From table III it is seen that most of the antibodies reacted with mesenchymal tumors yet apparently not with normal mesenchymal tissue. None of the antibodies reacted with lymhoid tissue or lymphocytes.

The reaction in breast carcinomas was characterized by marked heterogeneity. Thus, some of the antigens detected were present only in approximately one-fifth of the cases investigated, whereas the antigen detected by the antibody MAC 14/39 was present in 80% of the carcinomas.

It appears from table III that the antibody MAC 40/43 reacted with 59% of the breast carcinomas and that the antigen detected was not expressed in normal breast tissue.

The antibody MAC 40/43 was therefore characterized further, and the reaction patterns for this antibody is expressed in table IV.

Table 4: Reaction patterns of the human monoclonal antibody MAC 40/43

|                   | Normal | benign | malignant |                   | normal | benign | malignant |
|-------------------|--------|--------|-----------|-------------------|--------|--------|-----------|
| skin              | ++     |        | ++        | bladder + ureter  | ++     |        | ++        |
| testes            | +++    |        | +++       | liver             | -      |        | -         |
| lymph nodes       | -      |        | -         | ventricle         | -      |        | -         |
| portio + vagina   | -      |        | +         | intestine         | + -    |        |           |
| mesothelium       | ++     |        |           | colon + rectum    | -      |        | ++ (+)    |
| corpus + cervix   | -      |        | +         |                   |        |        |           |
| ovary             | -      |        | + -       | breast            | -      | -7/19  | +++       |
| prostate          | + -    |        | +         |                   |        | +(+)11/19 |        |
| thyroid           | -      |        |           |                   |        | +++ 1/19 |         |

From table IV it is seen that the reaction in benign tumors is much weaker than in malignant tumors with only 5% (1 of 19) of benign tumors exhibiting a strong reaction, whereas the rest express the antigen weakly or not at all. Moreover the table shown that the reaction pattern in colon/rectum is the same as in breast tissue, with heterogenuous reaction in malignant tumors, and only weak, if any reactions in benign and normal tissue.

Antigen characterization.

MCF-7 cells were washed twice in PBS and solubilized in 15 mM deoxycholate in 20 mM Tris-HCl, pH 8.0, containing 1 mM phenylmethylsulfonyl fluoride, for 30 min at 4°C. The suspension was centrifuged at 50.000 x g for 30 min and mixed 1:1 with sample buffer containing 5% $\beta$-mecaptoethanol, boiled for 5 min and applied to a 3.5%, 12.6% SDS-polyacrylamide gel system.

Electrophoretic transfer of proteins to nitrocellulose paper (0.2 $\mu$m Sartorius, West Germany) was performed using a JC semi-dry electroblotter (Jancos, Denmark) according to the manufacturer's instructions. After blotting, additional protein binding sites on the nitrocellulose paper were saturated by exposure to 2% Tween 20 in Tris/NaCl/Tween for 5 min. Carbohydrates were cleaved by mild periodate oxidation at an acid pH, according to Woodward et al. (J. Immunol.Meth. 78, (1985) 143-153). Briefly, strips were incubated with 10 mM sodium periodate in 50 mM Na acetate, pH 4.5, 1 h at 22°C and aldehyde groups were reduced by exposure to 50 mM sodium borohydride in PBS for 30 min at 22°C. Control strips were incubated with 50 mM Na acetate, pH 4.5, and 50 mM sodium borohydride. the strips were incubated with MAC 40/43 supernatant for 2 h at 22°C. After 4 washes at 5 min intervals, the strips were incubated with peroxidase-conjugated goat anti-human IgM (Tago, USA) diluted 1:3000 in Tris/NaCl/Tween for 1 h at 22°C. Finally, strips were washed 4 times with 50 mM Na acetate and the peroxidase activity was demonstrated using AEC as substrate.

After this treatment a band with an approximate Mr of 47,000 could be visualized. The reactivity diminished after treatment of MCF-7 cell components with 10 mM periodate, suggesting that the MAC 40/43 antigen is a glycoprotein.

## Claims

1. Human anticancer monoclonal antibodies synthesized by hybridoma cell lines produced by fusing human lymph node cells from regional axillary lymph nodes from a patient with carcinoma of the breast and a human cell line that is drug or temperature sensitive.

2. Human monoclonal antibodies as defined in Claim 1 wherein said human cell line is a myeloma-lymphoblastoid hybrid cell line.

3. Human monoclonal antibodies as defined in Claim 2 wherein said hybrid cell line is KR-12.

4. Human monoclonal antibodies as defined in Claim 3 wherein said cell line KR-12 is sensitive to growth in media containing the drugs hypoxanthine, aminopterin and thymidine.

5. Human monoclonal antibodies as defined in any one of Claims 1 to 4 wherein the monoclonal antibodies are of the IgM or IgG class of antibodies.

6. Human monoclonal antibodies produced from hybridoma, MAC 40/43.

7. Human monoclonal antibodies as defined in any of the Claims 1 to 6 in suitably labelled form for use in radioimmunoimaging.

8. Use of a human monoclonal antibody as defined in any one of the Claims 1 to 6 in a medicament.

9. Hybridoma cell lines that produce human monoclonal antibodies formed from fusing human lymph node cells from regional axillary lymph nodes from a patient with carcinoma of the breast with a human cell line that is drug or temperature sensitive such that the desired hybridoma cell lines may be isolated from the fused cell lines by selective drug or temperature treatment.

10. Hybridoma cell lines as defined in Claim 9 wherein said human cell line is a myeloma-lymphoblastoid hybrid cell line.

11. Hybridoma cell lines as defined in Claim 10 wherein said hybrid cell line is KR-12.

12. Hybridoma cell lines as defined in Claim 11 wherein the hybrid cell line KR-12 is sensitive to growth in media containing the drugs hypoxanthine, aminopterin and thymidine.

13. Hybridoma cell lines as defined in any one of Claims 9 to 12 which secrete antibodies of the IgM or IgG class of antibody.

14. Hybridoma, MAC 40/43.

15. A method for distinguishing cancer cells from normal cells comprising the steps of forming human-human hybridomas that secrete human IgM or IgG monoclonal antibody by fusing human regional lymph node cells from a patient with cancer of the breast, and a human cell line that is sensitive to growth in drug supplemented media, isolating said human monoclonal antibodies and combining them with said cancer cells and detecting the monoclonal antibody associated with said cancer cells.

16. A method as described in Claim 15 wherein detection of human monoclonal antibodies associated with said cancer cells is by immunochemical, or radioimmunochemical techniques.

17. An antigen containing an epitope which is recognized by the antibody produced by the hybridoma cell lines MAC 40/43 (IgM) or immunologically equivalent monoclonal antibodies.

18. The antigen of Claim 17, wherein the epitope is a specific glycoprotein moiety produced by the mammary carcinoma cell line MCF-7.

19. The antigen of Claim 17, wherein the epitope is an anti-idiotype antibody moiety or an immunological equivalent thereof.

20. A substantially purified glycoprotein moiety capable of binding specifically to the antibody produced by hybridoma MAC 40/43.

21. A diagnostic kit for the detection in serum, in tissue or tissue extracts of human mammary cancer comprising the monoclonal antibody of any of Claims 1 to 7.

22. The diagnostic kit of Claim 21 adapted for dot-blot analysis, an immunometric sandwich assay or a competitive assay.

23. The diagnostic kit of Claim 21 adapted for immunohistochemical localization of human mammary carcinoma cells.

24. A diagnostic kit comprising a carrier being compartmentalized to receive in close confinement therein two or more containers,
a first container comprising an antigen containing an epitope recognised by the antibody produced by hybridoma MAC 40/43, and
a second container comprising a detectably labelled, monoclonal antibody which binds an epitopic glycoprotein moiety specific for human mammary carcinoma, which moiety is produced by the cell line MCF-7.

25. The kit of Claim 24 wherein said antibody in said second container is produced by the hybridoma cell line MAC 40/43.